# EUROPEAN PATENT APPLICATION

(11) **EP 1 847 276 A1**
(43) Date of publication of application: **24.10.2007**
(21) Application number: 06008179.1
(22) Date of filing: 20.04.2006
(51) Int. Cl.: A61L 17/04

(54) **Twisted suture**

(71) Applicant: DSM IP Assets B.V., 6411 TE Heerlen (NL)
(72) Inventor: Snijder, Carina Sacha, 6137 JR Sittard (NL); Dirks, Christiaan Henri Peter, 3650 Dilsen (BE)
(74) Representative: Dorrestijn, Antoon

(57) **Abstract**

Suture comprising a suture member, the suture member comprising ultra high molecular weight polyolefin filaments, characterized in that the suture member comprising the ultra high molecular weight polyolefin filaments consists of two or more strands, the strands having been twisted together to form the member.

## Description

The invention relates to a suture comprising a suture member, the suture member comprising ultra high molecular weight polyolefin filaments.

In EP-A-561108 a suture is disclosed comprising ultra high molecular weight polyethylene. The suture comprises at least 7 fibres, that are spiral braided or formed as a hollow spiroid braid with the fibres forming a core within the braid.

In US2003/0139775 a suture is disclosed comprising ultra high molecular weight polyolefin fibres and another fibre, for example a polyester fibre. The suture includes a multifilament cover of the ultrahigh molecular weight polyethylene fibres braided with polyester or nylon fibres.

In WO 2004/082724 a suture is disclosed comprising a core of twisted ultra high molecular weight polyethylene fibres and a braided cover made from a different material, for example polyester. It is stated in the patent application that a woven braid is the standard in the industry for sutures.

Disadvantages of the known sutures are that their structure is complicated, so that it is laborative to produce the sutures. Furthermore there is need for sutures having improved properties.

Aim of the invention is to provide a suture that is simple to produce and yet shows good properties.

Surprisingly this object is obtained if the suture member comprising ultra high molecular weight polyolefin filaments consists of two or more strands, the strands having been twisted together to form the member.

It is very surprising that with such a suture comprising a member of such a rather simple structure very good results are obtained, whereas the known suture members comprising the ultra high molecular weight polyolefin filaments since a long time all have a complicated structure, such as a braided structure rather than a twisted structure and often have a separate core and sheath structure, the sheath being braided around the core.

The suture member of the suture according to the invention is very soft, well pliable and yet stronger than the known sutures having the braided structure.

Furthermore the suture member has a smooth surface and a round intersection, so that it slides very easy through tissue when repairing a wound.

Moreover it shows high knot strength.

The suture according to the invention may consist of the suture member, or it may next to the suture member comprise further components, for example a needle may be attached to the suture, or the suture may comprise a bone anchor.

High molecular weight polyolefin filaments are known to the skilled person. The filaments have an elongate body whose length dimension is greater than the transverse dimensions of width and thickness. It is preferred for the filaments to have a tensile strength of at least 1.2 GPa and a tensile modulus of at least 40 GPa.

Homopolymers and copolymers of polyethylene and polypropylene are particularly suitable as polyolefins for the production of the high molecular weight polyolefin fibres. Furthermore, the polyolefins used may contain small amounts of one or more other polymers, in particular other alkene-1-polymers.

Preferably the filaments are ultra high molecular weight polyethylene (UHMWPE) filaments, prepared by a gel spinning process, as for example described in EP 0205960 A, EP 0213208 A1, US 4413110, GB 2042414 A, EP 0200547 B1, EP 0472114 B1, WO 01/73173 A1, and Advanced Fiber Spinning Technology, Ed. T. Nakajima, Woodhead Publ. Ltd (1994), ISBN 1-855-73182-7, and references cited therein. Gel spinning is understood to include at least the steps of spinning at least one filament from a solution of ultra-high molecular weight polyethylene in a spin solvent; cooling the filament obtained to form a gel filament; removing at least partly the spin solvent from the gel filament; and drawing the filament in at least one drawing step before, during or after removing spin solvent. Suitable spin solvents include for example paraffins, mineral oil, kerosene or decalin. Spin solvent can be removed by evaporation, by extraction, or by a combination of evaporation and extraction routes. Such filaments are commercially available as Spectra^{®} or Dyneema^{®} grades.

Good results are obtained if the UHMWPE has an intrinsic viscosity (IV. as determined according to method PTC-179 (Hercules Inc. Rev. Apr. 29, 1982) at 135°C in decalin, with dissolution time of 16 hours, with anti-oxidant DBPC in an amount of 2 g/l solution, and the viscosity at different concentrations extrapolated to zero concentration) of above 5 dl/g. Particularly suitable is UHMWPE with IV of between about 8 and 40 dl/g, more preferably between 10 and 30, or 12 and 28, or between 15 and 25 dl/g. These ranges represent an optimum in polymer processability and filaments properties. Intrinsic viscosity is a measure for molar mass (also called molecular weight) that can more easily be determined than actual molar mass parameters like Mₙ and M_{w}. There are several empirical relations between IV and M_{w}, but such relation is highly dependent on molar mass distribution. Based on the equation M_{w} = 5.37 x 10⁴ [IV]^{1.37} (see EP 0504954 A1) an IV of 8 dl/g would be equivalent to Mw of about 930 kg/mol.

Preferably, the UHMWPE is a linear polyethylene with less than one branch or side chain per 100 carbon atoms, and preferably less than one side chain per 300 carbon atoms, a branch usually containing at least 10 carbon atoms. The linear polyethylene may further contain up to 5 mol% of one or more comonomers, such as alkenes like propylene, butene, pentene, 4-methylpentene or octene.

In a preferred embodiment, the UHMWPE contains a small amount of relatively small groups as side chains, preferably a C1-C4 alkyl group. It is found that a filament from UHMWPE with a certain amount of such groups show reduced creep behaviour. Too large a side chain, or too high an amount of side chains, however, negatively affects the processing and especially the drawing behaviour of the filaments. For this reason, the UHMWPE preferably contains methyl or ethyl side chains, more preferably methyl side chains. The UHMWPE therefore contains preferably at least 0.2, 0.3, 0.4 or 0.5 methyl or ethyl side chains. The amount of side chains is preferably at most 20, more preferably at most 10 per 1000 carbon atoms.

The UHMWPE can be a single polymer grade, but also a mixture of two or more different grades, e.g. differing in IV or molar mass distribution, and/or number of side chains.

The UHMWPE filaments may further contain usual amounts, generally less than 5 mass% of customary additives, such as anti-oxidants, thermal stabilizers, colorants, nucleating agents, flow promoters, catalyst residues etc.; as long as these components are suitable for the use in a surgical product. The UHMWPE filaments preferably contain less than 800 ppm of residual amounts of spin solvent, more preferably less than 500, 250, or even less than 100 ppm. The filaments may also contain other polymers, preferably polyolefinic polymers, like other polyethylenes, polypropylenes, or their copolymers, including rubbery copolymers like EPDM, EPR, etc. The amount of such other polymer is always lower than the amount of UHMWPE in the filament, and is preferably not more than 30 mass%, or more preferably not more than 20, 10 or 5 mass% of the UHMWPE filament.

A strand is the largest structural element in the suture member according to the invention. A strand may consist of a monofilament, a yarn or two or more assembled yarns. It is even possible that a strand consists of two or more sub-strands, that as such have the same structure as defined above for a strand. Preferably, the strand is a single multifilament yarn.

In case a strand contains more than one yarn, preferably the yarns are twisted together to form the strands in a direction that is opposite to the direction in which the strands are twisted together to form the suture member.

In case a strand contains sub-strands, preferably the sub-strands are twisted together to form the strands in a direction that is opposite to the direction in which the strands are twisted together to form the suture member. The opposite twist direction suppresses the unravelling of the suture element.

The suture member may consist of between 2 and 8 strands.

In one preferred embodiment the suture member consists of 2 strands, the strands having been twisted together to form the suture member. In that case the suture member can readily be knotted. This is an important property for sutures as during an operation many knots have to be made. Easy application of the knots helps the surgeon quick and accurately doing his job. In another preferred embodiment the suture member consists of 3 or 4 strands, the strands having been twisted together to form the suture member. In that case the suture slips very easy through tissue when the suture is applied for example when stitching a wound. The suture also has a very high strength.

A suitable size for the suture member of the suture according to the invention may be in the full USP range for sutures (e.g. 12-0 to 10). A USP value of 10 corresponds with a maximum diameter of 1.3 mm. In one preferred embodiment the suture member has a titer of between 25 and 500 dtex. In that case the suture is very suitable for cardiovascular operations. In another preferred embodiment the suture has a titer of between 500 and 3000 dtex. In that case the suture is very suitable for use in orthopaedic applications. In yet another preferred embodiment the suture member has a titer of between 3000 and 9000 dtex. In that case the suture is very suitable to be used in heavy orthopaedic applications.

The strands in the suture member have a titer (or linear density) that may vary widely, for example from 10 to 5000 dtex. For making thicker or heavier members preferably more strands are used, rather than thicker strands, to better control flexibility of the construction.

The suture member may in addition to the ultra high molecular polyolefin filaments comprise further components, for example compounds that provide some functional effect, like anti-microbial or anti-inflammatory action, or that further improve knotting performance. The amount of such other components is generally limited to at most 20 mass% (relative to total cable mass), preferably at most 10, more preferably at most 5 mass%.

The suture member of the suture according to the invention comprises preferably at least 50 mass% of the ultra high molecular weight polyolefin filaments. The ultra high molecular weight polyolefin filaments contribute most to the strength properties of the member. Furthermore the filaments enhance the sliding properties of the member through tissue. Therefore more preferably the suture member comprises at least 60 mass% of ultra high molecular weight polyolefin filaments, more preferably at least 70, 80 or at least 90 mass%. The suture member may further comprise other fibres, e.g. other biocompatible materials like polymers, to provide some other additional properties to the member, including improved knot slip behaviour or visual contrast. Such other fibres may be present in the form of one or more strands in the member. However, preferably each strand has the same composition, so that each strand comprises the same amount of the polyolefin filaments and of the other filaments. This ensures that the suture element has a homogeneous structure.

Suitable examples of other fibrous materials include filaments or staple fibres made from non-absorbable polymers like other polyolefins, fluoropolymers, or semi-aromatic polyesters like polyethylene terephthalate, absorbable polymers like aliphatic polyesters based on e.g. lactides.

Most preferably the suture member consists of the polyolefin filaments.

Preferably the suture member is treated at one or both of its ends to prevent unravelling of the suture member. Good results are obtained if the treatment is carried out over a length of between 10 and 40 mm at an end of the suture member. One very good way of treatment is a heat treatment, preferably while applying a tension to the suture member, so that the filaments at the place of treatment are at least partly molten together.

The conditions for the treatment, like temperature, residence time and tension level are selected to be such that the filaments will soften or even start to melt, but without loosing their crystalline orientation. This allows them to combine and adhere to each other at least at their surface. The temperature at which the process is carried out is preferably within the range from about 150° C up to about 157° C for gel spun ultra high molecular weight polyethylene filaments. Residence times during which the precursor is exposed to these temperatures may be within the range from about 6 seconds to about 150 seconds. The invention is further explained in the figures.
In Fig. 1 a scheme is given for the production of a twisted suture, comprising 4 strands. In Fig. 2a the S and the Z twist directions are represented.
In Fig. 2b a schematic representation is given of an intersection of the suture according to Fig. 1.

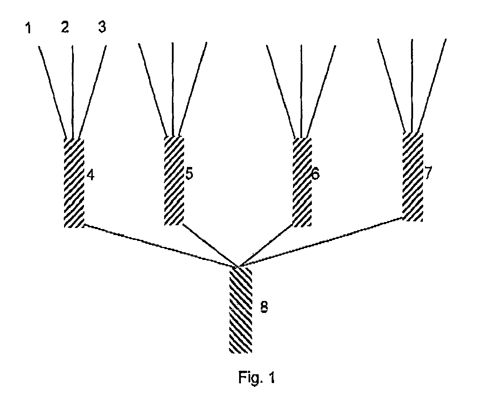

Fig. 1 shows a scheme for the production of a twisted suture according to the invention. Three yarns 1, 2 and 3 are twisted together in the Z-direction to form a strand 4. In parallel strands 5, 6 and 7 are twisted, in the same Z-direction. In this way each strand comprised 3 yarns. The four strands 4, 5, 6 and 7 are in a subsequent step twisted to form the twisted suture 8 according to the invention. The strands are twisted together in the opposite direction, the S-direction, to form the suture member.

In Fig. 2a twisted structure, being twisted in the S- and in the Z-direction are indicated. It is clear from the comparison that the S-direction is opposite to the Z-direction

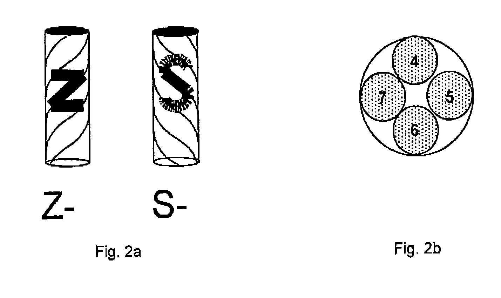

In Fig. 2b a schematic representation is given of an intersection, perpendicular to the length direction of the suture according to Fig. 1. Strands 4, 5, 6 and 7 are indicated.

### Comparative Experiment A

16 strands of a high molecular weight polethylene multifilament yarn of 110 dtex, sold by DSM Dyneema in the Netherlands, having a tenacity of 3.3 N/tex and tensile modulus of 94 N/tex, were braided on a Herzog braiding machine into a suture member with 24 picks per centimetre.

Properties of the suture member were determined applying following methods:
- Tensile properties: tensile strength (further indicated by tenacity), tensile modulus (further indicated by modulus) and elongation at break (further indicated by eab) are defined and determined with a procedure in accordance with ASTM D885M, using a nominal gauge length of the fibre of 500 mm, a crosshead speed of 50%/min, and Instron 2714 clamps, of type Fibre Grip D5618C for multifilament yarn. Strength of suture members was measured on a Zwick 1435 apparatus with Instron 1497K clamps. The suture member is preloaded at a tension of 50% of the linear breaking strength (Breaking strength is determined by a pre test). Subsequently the loading, at a speed of approx. 10 mm/min, is stopped until the residual tension in the suture member is 80% of the preload. Before the actual testing the preload is removed by decreasing the distance between the grips. On the basis of the measured stress-strain curve the modulus is determined as the gradient between 0.3 and 1 % strain. For calculation of the modulus and tenacity, the tensile forces measured are divided by the titer, as determined by weighing 10 metres of yarn or 1 metre of cable.
- The knot tenacity was analysed by making a single knot in the suture member. The suture member is clamped in the Zwick 1435 apparatus with Instron 1497K clamps in such a way that the knot is in the middle between the clamps. The suture member is preloaded at a tension of 50% of the breaking strength. Subsequently the loading is stopped until the residual tension on the knot is 80% of the preload (at this moment the knot is tensioned). Subsequently the knot is pulled until breakage. This experiment is repeated 5 times. The knot efficiency is calculated by dividing the tenacity of the single knot by the tenacity of the suture and multiplying the result with 100%. Results are collected in Table 1.

### Example 1

A suture member according to the invention was made by twisting together 3 strands consisting of high molecular weight polyethylene filaments. The strands had a titer of 440 dtex. The strands were twisted with 273 turns per meter in the Z-direction on a Saur Alma TT47 & Saur Alma TT06 twisting machine. Whereas the strands itself had been twisted with 465 turns per meter in the S-direction. Properties were determined as indicated above; results are collected in Table 1.

### Example 2

A suture member according to the invention was made by twisting together 3 strands consisting of high molecular weight polyethylene filaments. The strands had a titer of 880 dtex. The strands were twisted with 191 turns per meter in the Z-direction on a Saur Alma TT47 & Saur Alma TT06 twisting machine. Whereas the itself strands had been twisted with 331 turns per meter in the S-direction. Properties were determined as indicated above; results are collected in Table 1.

**Table 1**

| Sample | Titer (dtex) | | Theoretical strength suture (N, 100% eff.) | Analysed strength suture (N) | Efficiency linear strength (%) | Knot strength (N) |
|---|---|---|---|---|---|---|
| | Strand | Suture | | | | |
| Comp. Ex. 1 | 110 | 2129 | 707 | 291 | 41 | 123 |
| Ex. 1 | 440 | 1338 | 424 | 301 | 71 | 124 |
| Ex. 2 | 880 | 2818 | 860 | 580 | 67 | 238 |

As can be concluded from comparing comparative experiment A and example 1 the suture member according to the invention is about as strong as the conventional braided suture although the titer is much lower (37% less ultra high molecular weight polyethylene was used). Also the knot strength is equal.

Although the suture member of example 2 has somewhat higher titer than the suture member of comparative experiment A, the properties are on a much higher level.

## Claims

1. Suture comprising a suture member, the suture member comprising ultra high molecular weight polyolefin filaments, **characterized in that** the suture member comprising the ultra high molecular weight polyolefin filaments consists of two or more strands, the strands having been twisted together to form the member.

2. Suture according to claim 1, **characterized in that** the ultra high molecular weight polyolefin filaments are ultra high molecular weight polyethylene filaments made by a gel spinning process.

3. Suture according to any one of claims 1 or 2, **characterized in that** the suture comprises at least 50 mass% of the ultra high molecular weight polyolefin filaments.

4. Suture according to any one of claims 1 - 3, **characterized in that** the suture member consists of between 2 and 8 strands.

5. Suture according to any one of claims 1 - 3, **characterized in that** suture member consists of 2 strands, the strands having been twisted together to form the suture member.

6. Suture according to any one of claims 1 - 3, **characterized in that** the suture member consists of 3 or 4 strands, the strands having been twisted together to form the suture member

7. Suture member according to any one of claims 1 - 6, **characterized in that** the strands in the suture member have a titer of between 10 and 5000 dtex.

8. Suture member according to any one of claims 1 - 7, **characterized in that** the member is treated at one or both of its ends to prevent unraveling.

9. Suture member according to claim 8, **characterized in that** as treatment a heat treatment is applied.
